(19) Europäisches Patentamt
European Patent Office
Office européen des brevets

(11) Numéro de publication : 0 364 326 B1

(12) # FASCICULE DE BREVET EUROPEEN

(45) Date de publication du fascicule du brevet : **28.04.93 Bulletin 93/17**

(51) Int. Cl.[5] : **C07C 313/02,** C07C 69/74, A01N 53/00

(21) Numéro de dépôt : **89402684.8**

(22) Date de dépôt : **29.09.89**

(54) **Nouveax dérivés de l'acide 2,2-diméthyl 3-(1-hydroxy 2-sulfinoéthyl) cyclopropane carboxylique, leur procédé de préparation et leur application comme pesticides.**

(30) Priorité : **29.09.88 FR 8812740**

(43) Date de publication de la demande : **18.04.90 Bulletin 90/16**

(45) Mention de la délivrance du brevet : **28.04.93 Bulletin 93/17**

(84) Etats contractants désignés : **CH DE GB IT LI NL**

(56) Documents cités :
**EP-A- 0 187 674**
**EP-A- 0 217 342**
**EP-A- 0 269 514**
**GB-A- 2 099 810**

(73) Titulaire : **ROUSSEL-UCLAF**
**35, Boulevard des Invalides**
**F-75007 Paris (FR)**

(72) Inventeur : **Tessier, Jean**
**30, rue Jean Moulin**
**F-94300 Vincennes (FR)**
Inventeur : **Demassey, Jacques**
**Allée Saint Jacques Chalifert**
**F-77144 Montevrain (FR)**
Inventeur : **Demoute, Jean-Pierre**
**Canta Cigalo**
**F-13390 Auriol (FR)**

(74) Mandataire : **Tonnellier, Marie-José et al**
**111, route de Noisy B.P. no 9**
**F-93230 Romainville (FR)**

**EP 0 364 326 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de l'acide 2,2-diméthyl 3-[1-hydroxy 2-sulfino-éthyl] cyclopropane carboxylique, leur procédé de préparation et leur application comme pesticides. Des dérivés de l'acide 3-[1-méthylsulfonyloxy) éthyl] 2,2-diméthyl cyclopropane carboxylique sont décrits dans les demandes de brevet européen EP-A-269.514 et EP-A-217.342. L'invention a pour objet, sous toutes les formes isomères possibles, ainsi que les mélanges d'isomères, les composés répondant à la formule (I) :

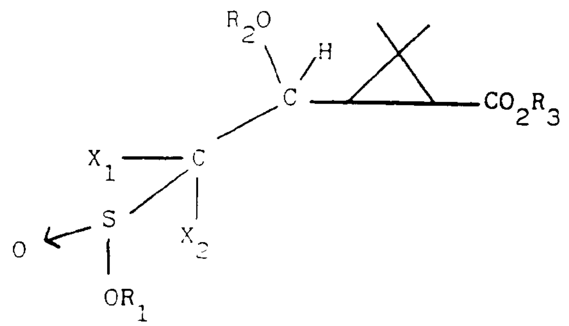

(I)

dans laquelle $R_1$ représente un atome d'hydrogène, ou un radical hydrocarboné renfermant jusqu'à 8 atomes de carbone linéaire ramifié ou cyclique saturé ou insaturé, $R_2$ représente un atome d'hydrogène, un radical hydrocarboné renfermant jusqu'à 8 atomes de carbone, linéaire, ramifié ou cyclique, saturé ou insaturé, ou un radical acyle renfermant jusqu'à 9 atomes de carbone linéaire, ramifié ou cyclique, saturé ou insaturé, $X_1$ et $X_2$ identiques ou différents représentent un atome de fluor, de chlore, de brome ou d'iode, un radical $CF_3$, C≡N, $CO_2R$ dans lequel R représente un radical alkyle linéaire renfermant de 1 à 8 atomes de carbone ou un radical

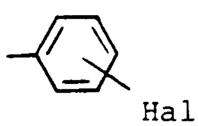

,

dans lequel Hal représente un atome de fluor, de chlore ou de brome, et $R_3$ représente un atome d'hydrogène, un radical alkyle linéaire saturé renfermant jusqu'à 8 atomes de carbone ou :

- soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylènedioxy et les atomes d'halogène,
- soit un groupement :

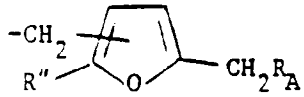

  dans lequel le substituant R" représente un atome d'hydrogène ou un radical méthyle et le substituant $R_A$ un aryle monocyclique ou un groupement -C CH et notamment un groupement 5-benzyl 3-furyl méthyle,
- soit un groupement :

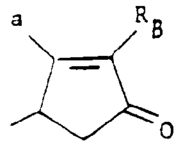

dans lequel a représente un atome d'hydrogène ou un radical méthyle et $R_B$ représente un radical organique aliphatique comportant de 2 à 6 atomes de carbone et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$, $-CH_2-C\ CH$,

- soit un groupement :

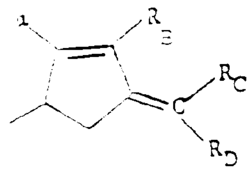

dans lequel a et $R_B$ conservent la même signification que précédemment, $R_C$ et $R_D$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

- soit un groupement :

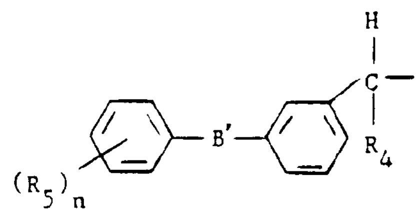

dans lequel B' représente un atome d'oxygène ou de soufre, un groupement

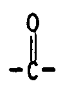

ou $-CH_2$ ou un groupement sulfoxyde ou un groupement sulfone et $R_4$ représente un atome d'hydrogène, un radical $-C{\equiv}N$, un radical méthyle, un radical $-CONH_2$, un radical $-CSNH_2$ ou un radical $-C{\equiv}H$, $R_5$ représente un atome d'hydrogène, un atome d'halogène ou un radical méthyle et n représente un nombre égal à 0,1 ou 2, et notamment le groupement 3-phénoxy benzyle, alpha-cyano 3-phénoxy benzyle, alpha-éthynyl 3-phénoxy benzyle, 3-benzoyl benzyle, 1-(3-phénoxy phényl) éthyle ou alpha-thioamido 3-phénoxy benzyle,

- soit un groupement :

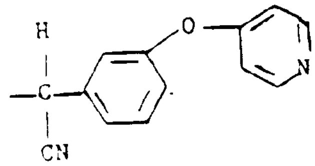

- soit un groupement :

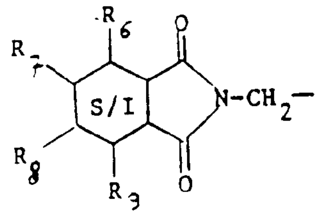

dans lequel les substituants $R_6$, $R_7$, $R_6$, $R_9$ représentent un atome d'hydrogène, un atome de chlore, ou un radical méthyle et dans lequel S/I symbolise un cycle aromatique ou un cycle analogue dihydro ou tétrahydro,

- soit un groupement :

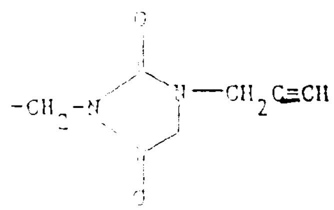

- soit un groupement

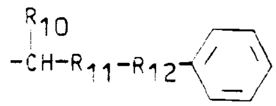

dans lequel $R_{10}$ représente un atome d'hydrogène ou un radical CN, $R_{12}$ représente un radical $-CH_2-$ ou un atome d'oxygène, $R_{11}$ représente un radical thiazolyle ou thiadiazolyle dont la liaison avec

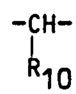

peut se trouver à l'une quelconque des positions disponibles, $R_{12}$ étant lié à $R_{11}$ par l'atome de carbone compris entre l'atome de soufre et un atome d'azote,

- soit un groupement :

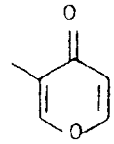

- soit un groupement :

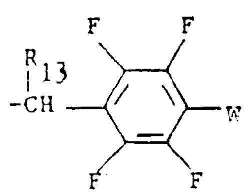

dans lequel $R_{13}$ représente un atome d'hydrogène ou un radical CN et W représente un atome d'hydrogène, un atome de fluor, un radical méthyle ou un radical méthoxy,

- soit un groupement :

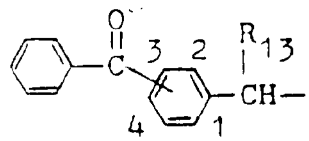

dans lequel $R_{13}$ est défini comme ci-dessus, et le radical benzoyle est lié aux positions 3 ou 4 du phényle,
- soit un groupement :

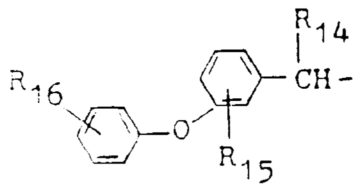

dans lequel $R_{14}$ représente un atome d'hydrogène, un radical méthyle éthynyle ou cyano et $R_{15}$ et $R_{16}$, différents, représentent un atome d'hydrogène, de fluor ou de brome,
- soit un groupement :

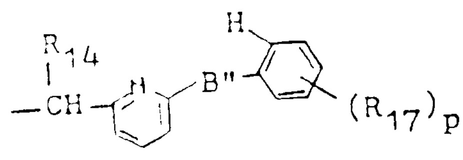

dans lequel $R_{14}$ est défini comme ci-dessus, chacun des $R_{17}$ représente indépendamment un atome d'hydrogène, un groupement alcoyle renfermant de 1 à 4 atomes de carbone, alcoxy renfermant de 1 à 4 atomes de carbone, alcoylthio renfermant de 1 à 4 atomes de carbone, alcoylsulfonyl renfermant de 1 à 4 atomes de carbone, trifluorométhyl, 3,4-méthylènedioxy, chloro, fluoro ou bromo, p représente un nombre égal à 0,1 ou 2 et B" représente un atome d'oxygène ou un atome de soufre,
- soit un groupement :

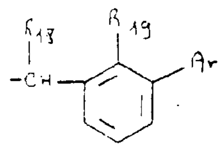

dans lequel $R_{18}$ représente un atome d'hydrogène, un radical méthyle, éthynyle ou cyano et $R_{19}$, différent, représente un atome d'hydrogène, de fluor ou de brome et Ar représente un radical aryle renfermant jusqu'à 14 atomes de carbone,
- soit un groupement :

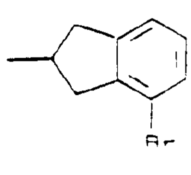

ou

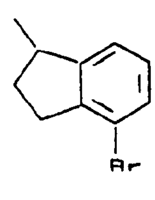

dans lesquels Ar est défini comme précédemment.

Parmi les composés préférés de l'invention, on peut citer les composés dans lesquels $R_3$ représente un

radical :

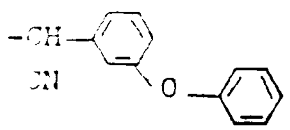

ou encore ceux dans lesquels $R_3$ représente un radical :

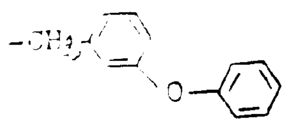

Les composés de formule I présentent plusieurs centres d'asymétrie, les carbones en 1 et 3 du cyclopropane, les carbones en 1' et le soufre de la chaîne latérale

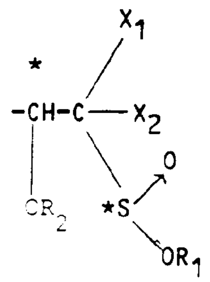

,

ils peuvent présenter également plusieurs centres d'asymétrie dans la partie $R_3$. L'invention a pour objet les différents stéréoisomères possibles ainsi que les mélanges de ces stéréoisomères.

Lorsque $R_1$ ou $R_3$ représente un radical hydrocarboné, il s'agit de préférence d'un radical méthyle, éthyle, n-propyle, isopropyle, n-butyle, isobutyle, terbutyle, d'un radical cyclopropyle, cyclobutyle, cyclopentyle ou cyclohexyle, d'un radical alcoyle, linéaire ou ramifié, portant un radical cyclique ou d'un radical cyclopropyle, cyclobutyle, cyclopentyle ou cyclohexyle substitué par un ou plusieurs radicaux alcoyles par exemple, le radical 1-méthylcyclobutyle, 1-méthyl cyclopentyle, 1-méthylcyclohexyle ou 2,2,3,3-tétraméthylcyclopropyle ou d'un radical vinyle ou 1, 1-diméthylallyle ou d'un radical acétylénique, comme, par exemple, le radical éthynyle ou propynyle.

Lorsque $R_2$ représente un radical acyle, il s'agit de préférence du radical $COCH_3$, $COC_2H_5$, $COC_6H_5$ ou $COCH_2C_6H_5$.

Lorsque $X_1$ ou $X_2$ représente un radical $CO_2R$, R représente de préférence un radical méthyle, éthyle, n-propyle, isopropyle, n-butyle, isobutyle ou terbutyle.

Lorsque $R_3$ représente un radical alkyle, il s'agit de préférence du radical méthyle, éthyle, n-propyle, isopropyle, butyle, isobutyle ou terbutyle. L'invention a plus particulièrement pour objet les composés de formule I dans lesquels $X_1$ et $X_2$ représentent chacun un atome de brome, ceux dans lesquels $R_1$ représente un atome d'hydrogène, et ceux dans lesquels $R_1$ représente un radical méthyle. Parmi les composés préférés de l'invention, on peut citer les composés dans lesquels $R_2$ représente un radical $COCH_3$ ainsi que ceux dans lesquels $R_2$ représente un radical méthyle. L'invention a notamment pour objet les composés de formule (I) dans lesquels la copule cyclopropanique est de structure 1R cis.

Parmi les composés préférés de l'invention, on peut citer tout particulièrement les produits dont la préparation est donnée dans la partie expérimentale exposée ci-après.

L'invention a également pour objet un procédé de préparation des composés de formule I caractérisé en ce que l'on soumet un composé de formule (II) :

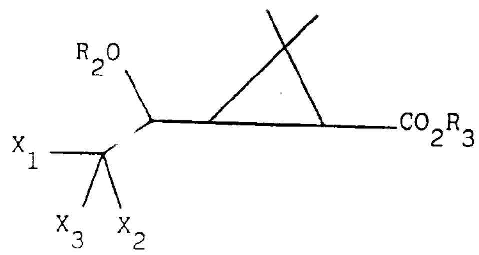

(II)

dans laquelle $X_1$, $X_2$, $R_2$ et $R_3$ conservent la même signification que précédemment et $X_3$ représente un atome de chlore, de brome ou d'iode, à l'action du dithionite de sodium en milieu aqueux pour obtenir le composé de formule $I_A$ correspondant à un produit de formule (I) dans laquelle $R_1$ représente un atome d'hydrogène

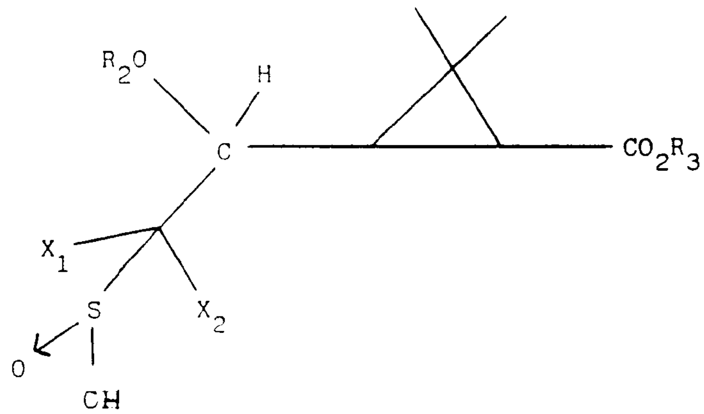

($I_A$)

que l'on soumet, si désiré, à l'action d'un agent d'étherification susceptible d'introduire le radical $R'_1$, $R'_1$ ayant la signification de $R_1$ à l'exception de la valeur hydrogène, pour obtenir le composé de formule $I_B$ :

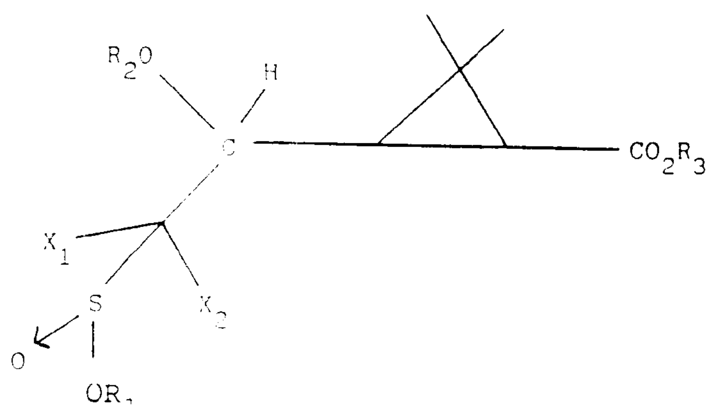

($I_B$)

correspondant à un produit de formule (I) dans laquelle $R_1$ est différent d'un atome d'hydrogène.

Dans un mode de réalisation préféré du procédé de l'invention, on fait réagir le composé de formule II et le dithionite de sodium en présence d'eau et d'un cosolvant pour solubiliser le produit de formule II, par exemple en présence d'eau et d'un alcool comme le méthanol, ou en présence d'eau et de tétrahydrofuranne ou de diméthylformamide.

Les composés de formule II utilisés comme produits de départ sont connus de manière générale et décrits dans EP-A-269. 514 et EP-A-217.342. Des exemples de préparation de composés de formule II sont donnés, ci-après, dans la partie expérimentale. Ces préparation peuvent être schématisées comme suit :

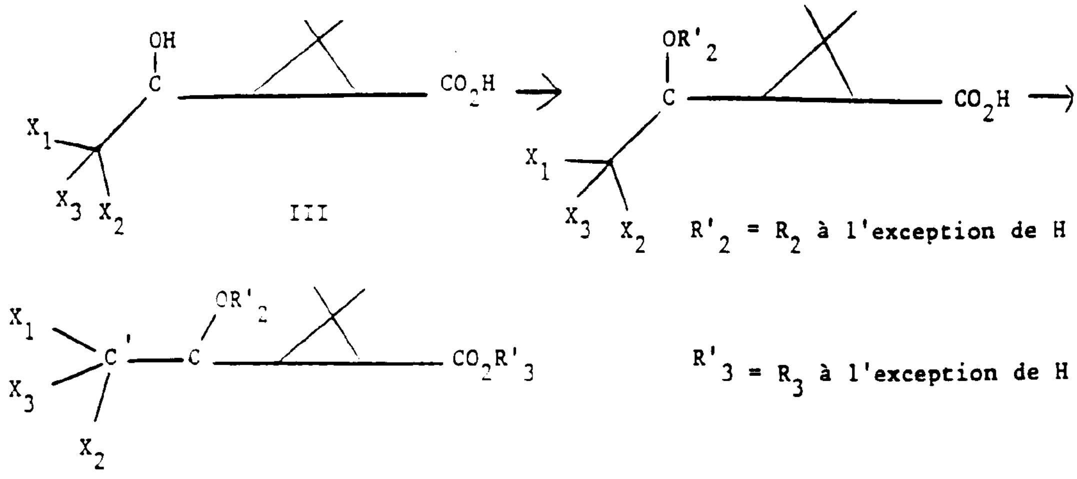


Il est clair que l'on peut d'abord estérifier le produit III pour obtenir le produit de formule :

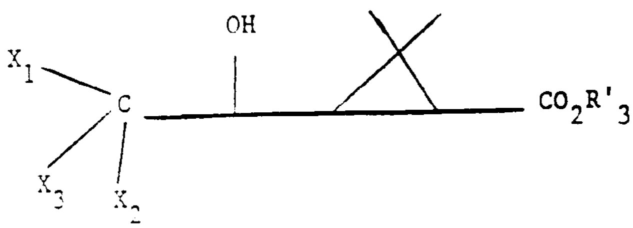

puis fonctionnaliser le groupement hydroxyle pour obtenir le produit :

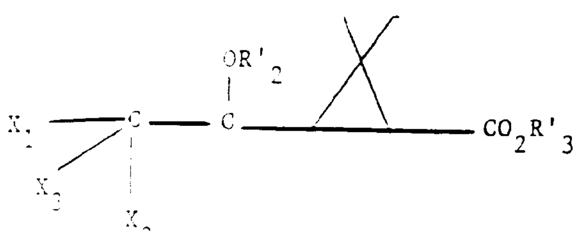

Les composés de formule III sont connus d'une manière générale et peuvent être préparés selon le procédé décrit dans le brevet français 2396006. Les composés de formule (I) présentent d'intéressantes propriétés pesticides : ils sont de plus solubles dans l'eau et peuvent être utilisés de façon systémique dans la lutte contre les parasites des végétaux.

Les composés de formule I trouvent leur utilisation dans la lutte contre les parasites ; il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a donc pour objet l'application des composés de formule (I) à la lutte contre les parasites des végétaux, les parasites des locaux et les parasites des animaux à sang chaud.

Les produits de formule (I) peuvent donc être utilisés notamment pour lutter contre les insectes dans le domaine agricole, pour lutter par exemple contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) peuvent aussi être utilisés pour lutter contre les acariens parasites des végétaux.

Les composés de formule (I) peuvent aussi être utilisés pour lutter contre les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus, ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits définis ci-dessus.

L'invention a notamment pour objet les compositions insecticides renfermant comme principe actif au moins l'un des produits définis ci-dessus.

Les compositions selon l'invention sont préparées selon les procédés usuels de l'industrie agrochimique ou de l'industrie vétérinaire ou de l'industrie des produits destinés à la nutrition animale.

Ces compositions peuvent se présenter sous forme de poudres, granulés, suspensions, émulsions, solutions, solutions pour aérosols, bandes combustibles, appats ou autres préparations employés classiquement pour l'utilisation de ce genre de composés.

Outre le principe actif, ces compositions contiennent, en générale, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active, est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles de Machilus Thumbergii), poudre de tige de pyrèthre, poudre de feuille de cèdre, poudre de bois (telle que la sciure de pin), amidon et poudre de coque de noix de coco. La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet, les compositions acaricides renfermant comme principe actif au moins des produits de formule (I) définie ci-dessus.

L'invention a également pour objet les compositions nématicides renfermant comme principe actif au moins des produits de formule (I) ci-dessus.

Les compositions insecticides selon l'invention, comme les compositions acaricides peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % ou des liquides pour pulvérisation foliaire contenant de 1 à 500 g/l de principe actif. On peut également employer des poudres pour poudrage foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

L'invention a également pour objet les compositions acaricides destinées à la lutte contre les acariens parasites des animaux à sang chaud, notamment contre les tiques et les gales, caractérisées en ce qu'elles renferment comme principe actif, au moins l'un des produits de formule (I) définie ci-dessus.

Lorsqu'il s'agit de lutter contre les parasites des animaux, les compositions peuvent être utilisées sous forme de spray, de bains, ou encore selon la méthode "pour-on".

Lorsqu'on utilise la méthode pour-on, on utilise de préférence des solutions renfermant de 0,5 g à 4 g de principe actif pour 100 cm3 de solution.

Lorsqu'il s'agit de lutter contre les acariens parasites des animaux, on incorpore très souvent les produits

de l'invention dans des compositions alimentaires en association avec un mélange nutritif adapté à l'alimentation animale. Le mélange nutritiel peut varier selon l'espèce animale, il peut renfermer des céréales, des sucres et des grains, de tourteaux de soja, d'arachide et de tournesol, des farines d'origine animale, par exemple des farines de poissons, des acides aminés de synthèse, des sels minéraux, des vitamines et des anti-oxydants.

L'invention a donc également pour objet les compositions destinées à l'alimentation animale, renfermant comme principe actif au moins l'un des produits de formule (I) telle que définie précédemment.

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3,4,5,6-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcool-alpha-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido-méthylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2,-tétrahaloéthyl) cyclopropane-1-carboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les associations selon l'invention présentent notamment l'intérêt, soit de permettre de combattre, par la polyvalence de leur action, une gamme de parasites, plus étendue, soit de manifester, dans certains cas, un effet de synergie.

Pour exalter l'activité biologique des produits de l'invention, on peut les additionner à des synergistes classiques utilisés en pareil cas tel le 1-(2,5,8-trioxadodécyl) 2-propyl 4,5-méthylènedioxy benzène (ou butoxyde de pipéronyle) ou la N-(2-éthylheptyl) bicyclo [2,2,1] 5-heptène-2,3-dicarboximide, ou le pipéronyl-bis-2-(2'-n-butoxyéthoxy) éthylacétal (ou tropital).

L'invention a pour objet les compositions pesticides définies précédemment, caractérisées en ce qu'elles renferment en outre un synergiste des pyréthrinoïdes.

**Exemple 1 : [1R[1alpha(S*),3alpha(R*)]]-3-[1-acétyloxy-2,2-dibromo-2-(méthoxy sulfinyl) éthyl]-2,2-diméthyl cyclopropane carboxylate de cyano (3-phénoxyphényl) méthyle.**

On ajoute à 20¤C environ 2,5 g de dithionite de sodium dissous dans 100 ml d'eau dans une solution de 7 g de produit préparé à la préparation 1 dans 100 $cm^3$ de méthanol. On ajoute 120 $cm^3$ de méthanol et 50 $cm^3$ de tétrahydrofuranne. On agite le mélange réactionnel pendant 30 minutes à 20¤C. On évapore les solvants sous pression réduite à 20-25¤C. On acidifie la phase aqueuse à l'aide d'une solution d'acide chlorhydrique concentrée jusqu'à obtention d'un pH de 1,5. On sature la phase aqueuse à l'aide de chlorure de sodium et extrait à l'acétate d'éthyle. On sèche et concentre à 25¤C sous pression réduite. On dissout le produit obtenu dans 50 $cm^3$ de chlorure de méthylène et ajoute 80 $cm^3$ d'une solution de diazométhane dans le chlorure de méthylène. On agite ensuite le mélange réactionnel pendant un quart d'heure à 25¤C et concentre. On purifie le produit brut obtenu par chromatographie sur silice en éluant par le mélange hexane-éther isopropylique (1-1). On sépare

le diastéréoisomère A $alpha_D$ = -49¤ ± 1¤ c=1 % $CHCl_3$

le diastéréoisomère B $alpha_D$ = +34,5¤ ± 1¤ c=1 % $CHCl_3$

**Exemple 2 : 1R[1alpha(S*),3alpha(R*)] 2,2-diméthyl 3-[1-acétyloxy-2,2-dibromo-2-(hydroxysulfinyl) éthyl] cyclopropane carboxylate de (3-phénoxyphényl) cyanométhyle.**

On dissout, à 5¤C environ, 2,96 g de produit préparé à la préparation 1 dans 30 $cm^3$ de tétrahydrofuranne, 10 $cm^3$ de méthanol et 10 $cm^3$ d'eau, on ajoute une solution de 1 g de dithionite de sodium dans 10 $cm^3$ d'eau. On laisse remonter la température à 20¤C et agite 45 minutes à cette température. On élimine la majorité des solvants sous pression réduite à 20¤C. On dilue par un peu d'eau pour obtenir une solution que l'on extrait à

l'éther éthylique. On refroidit la phase aqueuse à 0 ± 5¤C, acidifie par 14 mmoles d'acide chlorhydrique et extrait à l'éther éthylique. On sèche cette nouvelle phase éthérée, filtre et concentre sous pression réduite à 20¤C. On obtient 1,58 g de produit recherché.

**Example 3** : **1R[1alpha,3alpha(R*)] 2,2-diméthyl 3-[1-acétyloxy 2,2-dibromo 2-(hydroxysulfinyl) éthyl] cyclopropane carboxylate de (3-phénoxyphényl) méthyle.**

A une solution de 5,0 g de produit préparé à la préparation 2 dans un mélange de 80 $cm^3$ de tétrahydrofuranne et 20 $cm^3$ d'eau, à 5¤C, on ajoute 1,9 g de dithionite de sodium en solution dans 10 $cm^3$ d'eau. On laisse la température remonter à 20¤C environ et on agite environ trois quarts d'heure à cette température. On élimine le tétrahydrofuranne sous pression réduite à 20¤C. On dilue le résidu à l'eau pour obtenir une solution que l'on extrait à l'éther éthylique. On décante la phase aqueuse, l'acidifie par 20 mmoles d'acide chlorhydrique et l'extrait à l'éther éthylique. On sèche, filtre et concentre à sec sous pression réduite à 20¤C. On obtient 3,08 g de produit recherché.

**Exemple 4** : **1R[1alpha,3alpha(R*)] 2,2-diméthyl 3-[1-méthoxy 2,2-dibromo 2-(hydroxysulfinyl) éthyl] cyclopropane carboxylate de (3-phénoxyphényl) méthyle.**

On dissout sous agitation magnétique et atmosphère d'azote 2,95 g d'acide 1R cis 3(2,2,2-tribromo 1R méthoxy éthyl) 2,2-diméthyl cyclopropane carboxylique et 1,6 g d'alcool métaphénoxybenzylique dans 50 $cm^3$ de Chlorure de méthylène. On ajoute à 0¤ ± 5¤C 0,2 g de diméthylaminopyridine, et 1,65 g de dicyclohexylcarbodiimide, on amène à 20¤C et agite pendant 30 minutes. On élimine l'insoluble par filtration. On concentre le filtrat sous pression réduite à 40¤C. On chromatographie sur silice en éluant par le mélange hexane-acétate d'éthyle 95-5. On obtient ainsi 3,33 g d'ester de (3-phénoxyphényl) méthyle de l'acide de départ que l'on dissout dans un mélange de 30 $cm^3$ de tétrahydrofuranne, 10 $cm^3$ de méthanol, 10 $cm^3$ d'eau. On ajoute ensuite à 0 ± 5¤C 1,04 g de dithionite de sodium en solution dans 10 $cm^3$ d'eau. On laisse remonter la température à 20¤C et agite environ 45 minutes à cette température. On élimine les solvants sous pression réduite à 20¤C. On dilue le résidu à l'eau, refroidit à 0 ± 5¤C, acidifie à pH 1 par l'acide chlorhydrique et extrait à l'éther éthylique. On sèche la phase éthérée, filtre et concentre sous pression réduite à 20¤C.
On obtient ainsi 2,12 g de produit recherché.

**Exemple 5** : **1R[1alpha(S*),3alpha(R*)] 2,2-diméthyl 3-[1-méthoxy 2,2-dibromo 2-(hydroxysulfinyl) éthyl] cyclopropane carboxylate de (3-phénoxyphényl) cyanométhyle.**

On dissout sous agitation magnétique 2,87 g de 1R cis 3-(2,2,2-tribromo 1R-méthoxyéthyl) 2,2-diméthyl cyclopropane carboxylate de S cyano 3-phénoxyphényl) méthyle, dans une solution renfermant 25 $cm^3$ de tétrahydrofuranne, 20 $cm^3$ de méthanol et 10 $cm^3$ d'eau. On ajoute une solution renfermant 0,94 g de dithionite de sodium dans 8 $cm^3$ d'eau sans dépasser 20¤C. On agite la solution pendant 30 minutes à 20¤C. On élimine le méthanol et le tétrahydrofuranne sous pression réduite. On dilue le résidu à l'eau et extrait à l'éther éthylique. On décante la phase aqueuse, la refroidit à 0 ± 5¤C et l'acidifie par 10 mmoles d'acide chlorhydrique. On extrait à l'éther éthylique, sèche, filtre et concentre sous pression réduite. On obtient ainsi 2,35 g de produit recherché.

**Exemple 6** : **1R-[1alpha(S*),3alpha(R*)] 2,2-diméthyl 3-(1-méthoxy 2,2-dibromo 2-méthoxy sulfinyl éthyl) cyclopropane carboxylate de (3-phénoxyphényl) cyanométhyle.**

On dissout 2,52 g de produit préparé à l'exemple 5 dans 20 $cm^3$ de chlorure de méthylène. On ajoute à 0¤ ± 5¤C, une solution de diazométhane dans le chlorure de méthylène. On ajoute une goutte d'acide acétique. On concentre la solution sous pression réduite à 20¤C. on chromatographie sur silice en éluant par le 1,2-dichloroéthane.
On obtient ainsi 1,94 g de produit recherché.

**Préparation 1 : 1R cis 3-(2,2,2-tribromo 1R acétoxyéthyl) 2,2-diméthyl cyclopropane carboxylate de S cyano (3-phénoxyphényl) méthyle.**

**Stade A :** acide 1R cis 3-(2,2,2-tribromo 1R acétoxyéthyl 2,2-diméthyl cyclopropane carboxylique.

On ajoute à -50¤C une solution de 5,75 g de terbutylate de potassium, 20 $cm^3$ de tétrahydrofuranne et 30 $cm^3$ d'alcool terbutylique dans une solution renfermant 10 g d'acide 1R cis 2,2-diméthyl 2-(1-hydroxy 2',2',2'-tribromoéthyl) cyclopropane carboxylique et 30 $cm^3$ de tétrahydrofuranne. On agite trente minutes à -50¤C et ajoute 2,4 $cm^3$ d'anhydride acétique. On agite et laisse remonter la température à -20¤C. On agite pendant 50 heures à -20¤C et coule la suspension dans l'acide chlorhydrique 2N. On extrait au chlorure de méthylène, lave à l'eau, sèche et filtre. On amène le filtrat à sec. On obtient 9,7 g d'un produit que l'on verse dans une solution aqueuse de bicarbonate de sodium . On agite le mélange réactionnel pendant 16 heures à 20¤C. On essore le cristallisat et le lave à l'eau. La phase aqueuse est acidifiée à pH1 avec de l'acide chlorhydrique. On extrait avec du chlorure de méthylène, lave à l'eau, sèche et filtre. On amène le filtrat à sec.
On obtient 3,3 g de produit recherché F=178¤C.

**Stade B :** 1R[1alpha(S*),3alpha(R*)] 2,2-diméthyl 3-[1-acétyloxy 2,2,2-tribromoéthyl] cyclopropane carboxylate de 3-phénoxyphényl cyanométhyle.

On dissout 11,64 g d'acide préparé au stade A et 6 g d'alcool (S) alpha cyano (3-phénoxy benzylique dans 75 $cm^3$ de chlorure de méthylène à +10¤C. On ajoute une solution de 5,5 g de dicyclohexylcarbodiimide, 0,32 g de diméthyl amino pyridine dans 50 $cm^3$ de chlorure de méthylène. On ramène à 20¤C et agite 18 heures. L'insoluble est éliminé par filtration. Le filtrat est concentré sous pression réduite, à 40¤C. On chromatographie sur silice en éluant par un mélange hexane-éther isopropylique 7-3.
On obtient ainsi 13 g de produit recherché.

**Préparation 2 : 1R cis 3-(2,2,2-tribromo 1R acétoxy éthyl) 2,2-diméthyl cyclopropane carboxylate de 3-(phénoxyphényl) méthyle.**

**Stade A :** 1R cis 3-(2,2,2-tribromo 1R hydroxyéthyl) 2,2-diméthyl cyclopropane carboxylate de 3-(phénoxyphényl) méthyle.

On introduit à 20¤C 10 g d'acide 1R cis 2,2-diméthyl 2-(1-hydroxy 2',2',2'-tribromoéthyl) cyclopropane carboxylique dans 200 $cm^3$ de benzene anhydre. On chauffe la suspension au reflux du benzène et distille pour entrainer l'eau. En fin de distillation, on amène à sec sous pression réduite. On obtient un résidu que l'on reprend dans 40 $cm^3$ de diméthylformamide anhydre. On refroidit la solution obtenue à -60¤C et ajoute 1,2 g d'hydrure de sodium à 50 %. On laisse remonter la température à 20¤C. On agite ensuite à 20¤C pendant 30 minutes. On ajoute 5,4 g de chlorure de métaphénoxybenzyle et agite le mélange réactionnel à 20¤C pendant 48 heures. On coule la suspension obtenue dans l'acide chlorhydrique 2N. On agite pendant 10 minutes et extrait au benzene. On lave à l'eau. On sèche, filtre. On amène le filtrat à sec sous pression réduite. On obtient 14,5 g de produit que l'on chromatographie sur silice en éluant par le mélange cyclohexane-acétate d'éthyl 8-2 recristallise le produit dans l'éther isopropylique et l'éther de pétrole (Eb : 60-80¤C).
On obtient 10,35 g de produit recherché F = 50¤C.

**Stade B :** 1R cis 3-(2,2,2-tribromo 1R acétoxy éthyl) 2,2-diméthyl cyclopropane carboxylate de 3-(phénoxyphényl) méthyle.

On agite à 20¤C pendant 2 jours une solution renfermant 1 g de produit préparé au stade A, 2 $cm^3$ de pyridine anhydre et 2 $cm^3$ d'anhydride acétique. On coule la solution dans de l'acide chlorhydrique 2N et agite 1 heure à 20¤C. On extrait au benzene. On lave la solution benzenique à l'eau. On sèche la phase organique. On filtre et l'amène à sec. On obtient 0,782 g de produit brut recherché que l'on chromatographie sur silice en éluant par le mélange cyclohexane-acétate d'éthyle 9-1.
On obtient ainsi le produit recherché.

**Préparation 3 : acide 1R cis 3-(2,2,2-tribromo 1R méthoxyéthyl) 2,2-diméthyl cyclopropane carboxylique.**

On ajoute à -20¤C 5,75 g de terbutylate de potassium, 20 $cm^3$ de tétrahydrofuranne et 30 $cm^3$ d'alcool terbutylique dans une solution renfermant 10 g d'acide 1R cis 2,2-diméthyl 2-(1-hydroxy 2',2',2'-tribromoéthyl) cyclopropane carboxylique dans 40 $cm^3$ de tétrahydrofuranne. On agite le mélange réactionnel pendant 30 minutes à -20¤C et ajoute 2,5 $cm^3$ de sulfate de méthyle et 10 $cm^3$ de tétrahydrofuranne. On agite pendant 17 heures à -20¤C. On verse dans un mélange d'eau et d'acide chlorhydrique. On agite à 20¤C pendant 10 minutes ; on extrait à l'éther éthylique, lave la phase organique à l'eau jusqu'à neutralité. On sèche, filtre, et amène à sec le filtrat sous pression réduite à 40¤C. On recristallise le produit obtenu dans le benzene, essore, lave les cristaux obtenus et sèche.

On obtient 3,7 g de produit recherché F=180¤C.

**Préparation 4 : 1R[1alpha(S*),3alpha(R*)] 2,2-diméthyl 3-(1-méthoxy 2,2,2-tribromoéthyl] cyclopropane carboxylate de 3-phénoxyphényl cyano méthyle.**

On dissout 7,1 g d'acide préparé à la préparation 3 et 4,5 g d'alcool (3-phénoxyphényl) (1S) cyano méthyle dans 100 $cm^3$ de chlorure de méthylène à 0 ± 5¤C. On ajoute 0,2 g de diméthylaminopyridine, 4,1 g de dicyclohexyl carbodiimide, ramène à 20¤C et agite 1 heure. L'insoluble est éliminé par filtration. Le filtrat est concentré sous pression réduite à 40¤C. On chromatographie sur silice en éluant par un mélange hexane-acétate d'éthyle 9-1.
On obtient ainsi 10,32 g de produit recherché.

**Exemple 7 : préparation d'un concentré soluble.**

On effectue un mélange homogène de :

| | |
|---|---|
| Produit de l'exemple 2 | 0,25 g |
| Butoxy de pipéronyle | 1,00 g |
| Tween 80 | 0,25 g |
| Topanol A | 0,1 g |
| Eau | 98,4 g |

**Exemple 8 : préparation d'un concentré émulsifiable.**

On mélange intimement :

| | |
|---|---|
| Produit de l'exemple 3 | 0,015 g |
| Butoxyde pipéronyle | 0,5 g |
| Topanol A | 0,1 g |
| Tween 80 | 3,5 g |
| Xylène | 95,885 g |

**Exemple 9 : préparation d'un concentré émulsifiable.**

On effectue un mélange homogène de :

| | |
|---|---|
| Produit de l'exemple 4 | 1,5 g |
| Tween 80 | 20,00 g |
| Topanol A | 0,1 g |
| Xylène | 78,4 g |

**Exemple 10 : préparation d'une composition fumigène.**

On mélange d'une façon homogène :

| | |
|---|---|
| Produit de l'exemple 5 | 0,25 g |
| Poudre de tabu | 25,00 g |
| Poudre de feuille de cèdre | 40,00 g |
| Poudre de bois de pin | 33,75 g |
| Vert brillant | 0,5 g |
| p-nitrophénol | 0,5 g |

**Etude Biologique**

1. Etude de l'activité létale sur mouche domestique

Les insectes tests sont des mouches domestiques femelles âgées de 4 à 5 jours. On opère par pulvérisation topique de 1 microlitre de solution acétonique sur le thorax dorsal des insectes à l'aide du micromanipulateur d'Arnold.

On utilise 50 individus par traitement. On effectue le contrôle de mortalité 24 heures après traitement.

Les résultats obtenus exprimés en DL 50 ou dose en nanogrammes par individu nécessaire pour tuer 50 % des insectes, sont les suivants :

| Composés de l'exemple | DL 50 ng/insecte |
| --- | --- |
| 2 | 7,78 |
| 5 | 7,3 |

Conclusion :

Les produits de l'invention sont doués d'un très bon effet létal sur mouches domestiques.

2. Etude de l'effet létal sur larves de Spodoptera Littoralis

Les essais sont effectués par application topique d'une solution acétonique à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24¤C et 65 % d'humidité relative. Après traitement, les individus sont placés sur un milieu nutritif artificiel (milieu de Poitout).

On effectue le contrôle des mortalités 48 heures après traitement.

Les résultats expérimentaux obtenus sont résumés dans le tableau suivant :

| Composés de l'exemple | DL 50 ng/insecte |
| --- | --- |
| 2 | 25,3 |
| 3 | 27,26 |
| 4 | 28,87 |
| 5 | 7,27 |

3. Etude de l'effet létal sur Aphis Cracivora

On utilise des adultes après 7 jours et l'on emploie 10 Aphis par concentration utilisée. On utilise une méthode de contact-ingestion. On effectue le traitement au pistolet de Fisher d'une feuille de fève qu'l'on dépose dans une boîte de Petri en matière plastique sur une rondelle de papier humidifiée. Le traitement est effectué à l'aide de 2 ml de solution acétonique de produit à tester (1 ml par face de feuille). L'infestation par insecte est effectuée après séchage de la feuille. On maintient les insectes en contact avec la feuille pendant une heure. On place les insectes sur des feuilles non traitées et contrôle la mortalité au bout de 24 heures.

Les résultats expérimentaux obtenus sont résumés dans le tableau suivant :

| Composé de l'exemple | DL 50 mg/litre |
|---|---|
| 3 | 3,6 |

## Revendications

1. Sous toutes les formes isomères possibles, ainsi que les mélanges d'isomères, les composés répondant à la formule (I) :

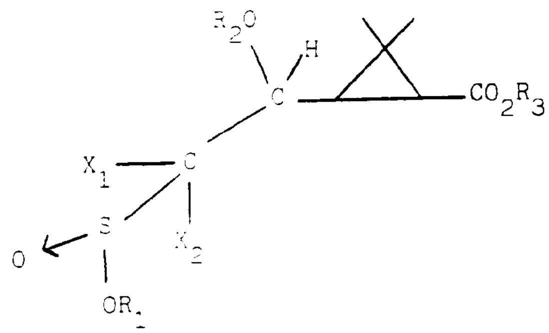

(I)

dans laquelle $R_1$ représente un atome d'hydrogène, ou un radical hydrocarboné renfermant jusqu'à 8 atomes de carbone linéaire ramifié ou cyclique saturé ou insaturé, $R_2$ représente un atome d'hydrogène, un radical hydrocarboné renfermant jusqu'à 8 atomes de carbone linéaire ramifié ou cyclique, saturé ou insaturé, ou un radical acyle renfermant jusqu'à 9 atomes de carbone, linéaire, ramifié ou cyclique, saturé ou insaturé, $X_1$ et $X_2$ identiques ou différents représentent un atome de fluor, de chlore, de brome ou d'iode, un radical $CF_3$, C≡N, $CO_2R$ dans lequel R représente un radical alkyle linéaire renfermant de 1 à 8 atomes de carbone ou un radical

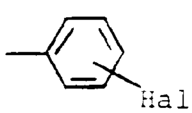

,

dans lequel Hal représente un atome de fluor, de chlore ou de brome, et $R_3$ représente un atome d'hydrogène, un radical alkyle linéaire saturé renfermant jusqu'à 8 atomes de carbone ou :

- soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylènedioxy et les atomes d'halogène,
- soit un groupement :

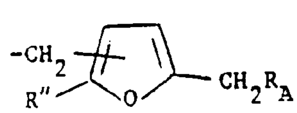

dans lequel le substituant R" représente un atome d'hydrogène ou un radical méthyle et le substituant $R_A$ un aryle monocyclique ou un groupement -C≡CH et notamment un groupement 5-benzyl 3-furyl méthyle,

- soit un groupement :

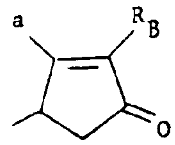

dans lequel a représente un atome d'hydrogène ou un radical méthyle et $R_B$ représente un radical organique aliphatique comportant de 2 à 6 atomes de carbone et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$, $-CH_2-C≡H$,

- soit un groupement :

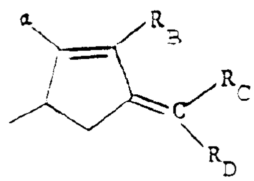

dans lequel a et $R_B$ conservent la même signification que précédemment, $R_C$ et $R_D$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,

- soit un groupement :

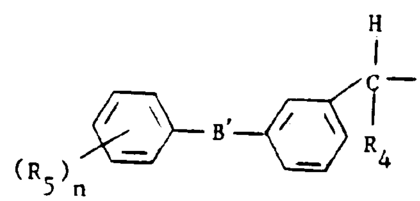

dans lequel B' représente un atome d'oxygène ou de soufre, un groupement

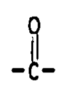

ou $-CH_2$ ou un groupement sulfoxyde ou un groupement sulfone et $R_4$ représente un atome d'hydrogène, un radical -C≡N, un radical méthyle, un radical $-CONH_2$, un radical $-CSNH_2$ ou un radical -C≡CH, $R_5$ représente un atome d'hydrogène, un atome d'halogène ou un radical méthyle et n représente un nombre égal à 0,1 ou 2, et notamment le groupement 3-phénoxy benzyle, alpha-cyano 3-phénoxy benzyle, alpha-éthynyl 3-phénoxy benzyle, 3-benzoyl benzyle, 1-(3-phénoxy phényl) éthyle ou alpha-thioamido 3-phénoxy benzyle,

- soit un groupement :

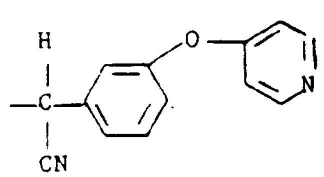

- soit un groupement :

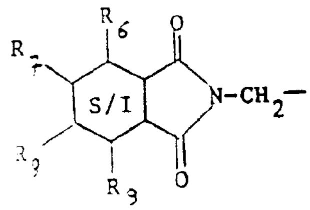

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

- soit un groupement

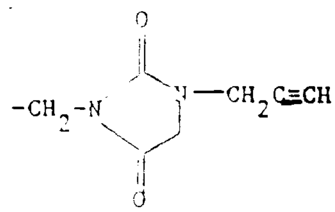

- soit un groupement

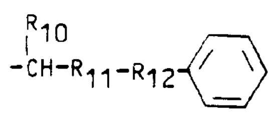

dans lequel $R_{10}$ représente un atome d'hydrogène ou un radical CN, $R_{12}$ représente un radical $-CH_2-$ ou un atome d'oxygène, $R_{11}$ représente un radical thiazolyle ou thiadiazolyle dont la liaison avec

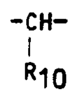

peut se trouver à l'une quelconque des positions disponibles, $R_{12}$ étant lié à $R_{11}$ par l'atome de carbone compris entre l'atome de soufre et un atome d'azote,

- soit un groupement :

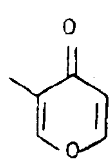

- soit un groupement :

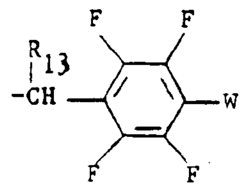

dans lequel $R_{13}$ représente un atome d'hydrogène ou un radical CN et W représente un atome d'hydrogène, un atome de fluor, un radical méthyle ou un radical méthoxy,

- soit un groupement :

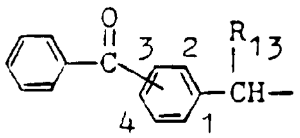

dans lequel $R_{13}$ est défini comme ci-dessus, et le radical benzoyle est lié aux positions 3 ou 4 du phényle,

- soit un groupement :

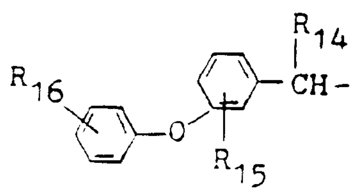

dans lequel $R_{14}$ représente un atome d'hydrogène, un radical méthyle éthynyle ou cyano et $R_{15}$ et $R_{16}$, différents, représentent un atome d'hydrogène, de fluor ou de brome,

- soit un groupement :

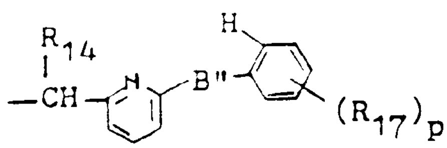

dans lequel $R_{14}$ est défini comme ci-dessus, chacun des $R_{17}$ représente indépendamment un atome d'hydrogène, un groupement alcoyle renfermant de 1 à 4 atomes de carbone, alcoxy renfermant de 1 à 4 atomes de carbone, alcoylthio renfermant de 1 à 4 atomes de carbone, alcoylsulfonyl renfermant de 1 à 4 atomes de carbone, trifluorométhyl, 3,4-méthylènedioxy, chloro, fluoro ou bromo, p représente un nombre égal à 0,1 ou 2 et B" représente un atome d'oxygène ou un atome de soufre,

- soit un groupement :

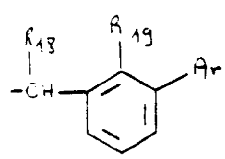

dans lequel $R_{18}$ représente un atome d'hydrogène, un radical méthyle, éthynyle ou cyano et $R_{18}$, différent, représente un atone d'hydrogène, de fluor ou de brome et Ar représente un radical aryle renfermant jusqu'à 14 atomes de carbone,

- soit un groupement :

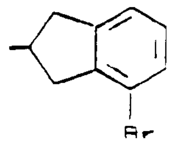

ou

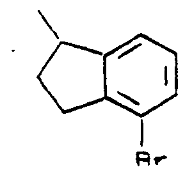

dans lesquels Ar est défini comme précédemment.

2. Les composés de formule I tels que définis à la revendication 1 dans lesquels $X_1$ et $X_2$ représentent chacun un atome de brome.

3. Les composés de formule I tels que définis à la revendication 1 ou 2, dans lesquels $R_1$ représente un atome d'hydrogène.

4. Les composés de formule I tels que définis à la revendication 1 ou 2, dans lesquels $R_1$ représente un radical méthyle.

5. Les composés de formule I tels que définis à l'une quelconque des revendications 1 à 4 dans lesquels $R_2$ représente un radical $COCH_3$.

6. Les composés de formule I tels que définis à l'une quelconque des revendications 1 à 4 dans lesquels $R_2$ représente un radical méthyle.

7. Les composés de formule I tels que définis à l'une quelconque des revendications 1 à 6 dans lesquels la copule cyclopropanique est de structure 1R cis.

8. Les composés de formule I définis à la revendication 1, dans lesquels $R_3$ représente un radical :

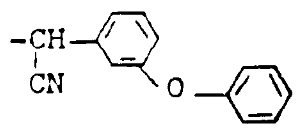

9. Les composés de formule I définis à la revendication 8, dans lesquels $R_3$ représente un radical :

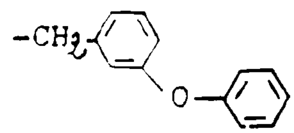

10. Procédé de préparation des composés de formule I tels que définis à l'une quelconque des revendications 1 à 9 , caractérisé en ce que l'on soumet un composé de formule (II) :

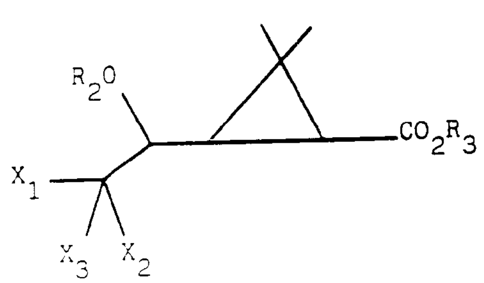

(II)

dans laquelle $X_1$, $X_2$, $R_2$ et $R_3$ conservent la même signification que précédemment et $X_3$ représente un atome de chlore, de brome ou d'iode, à l'action du dithionite de sodium en milieu aqueux pour obtenir le composé de formule $I_A$ correspondant à un produit de formule (I) dans laquelle $R_1$ représente un atome

d'hydrogène

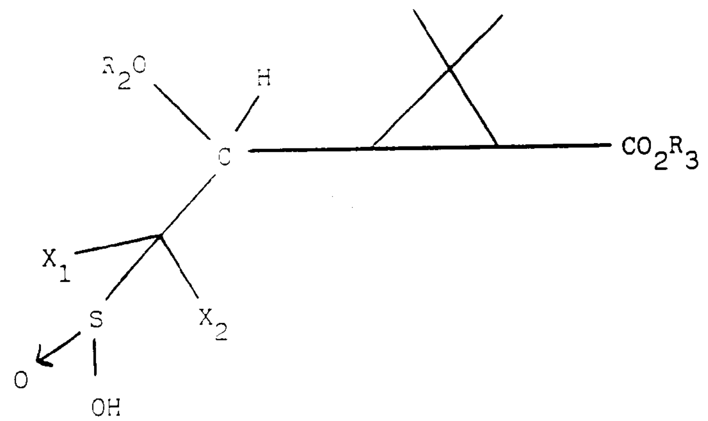

$(I_A)$

que l'on soumet, si désiré, à l'action d'un agent d'étherification susceptible d'introduire le radical $R'_1$, $R'_1$ ayant la signification de $R_1$ à l'exception de la valeur hydrogène, pour obtenir le composé de formule $I_B$ :

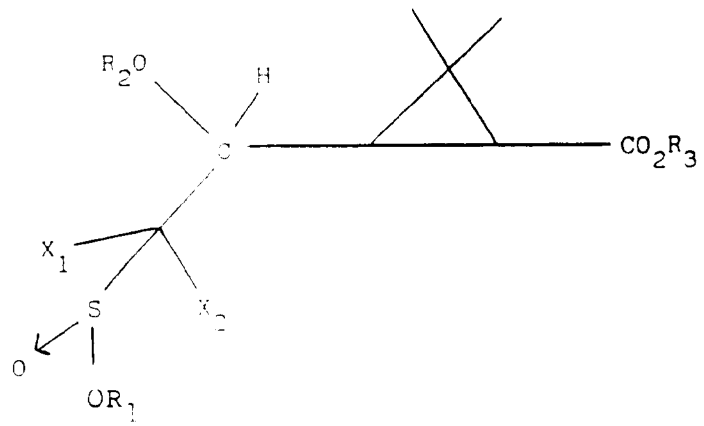

$(I_B)$

correspondant à un produit de formule (I) dans laquelle $R_1$ est différent d'un atome d'hydrogène.

**11.** Application des composés de formule I tels que définis à l'une des revendications 1 à 9 , à la lutte contre les parasites des végétaux et des animaux et les parasites des locaux.

**12.** Les compositions destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 9,

**13.** Les compositions insecticides renfermant comme principe actif au moins l'un des produits définis à l'une quelconque des revendications 1 à 9.

**14.** Les compositions acaricides renfermant comme principe actif au moins l'un des produits définis à l'une quelconque des revendications 1 à 9 .

**15.** Les compositions nématicides renfermant comme principe actif au moins l'un des produits définis à l'une quelconque des revendications 1 à 9 .

**16.** Les compositions acaricides destinées à la lutte contre les parasites des animaux à sang chaud, notamment contre les tiques et les gales, caractérisées en ce qu'elles renferment comme principe actif, au moins l'un des produits définis à l'une quelconque des revendications 1 à 9.

**17.** Les compositions destinées à l'alimentation animale renfermant comme principe actif au moins l'un des produits définis à l'une quelconque des revendications 1 à 9 .

18. Associations douées d'activité insecticide, acaricide ou nématicide, caractérisées en ce qu'elles contiennent comme matière active, d'une part un au moins des composés de formule générale (I) et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3,4,5,6-tétrahydrothiophénylidène méthyl) cyclopropane-1-carboxylique, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzylique d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzylique des acides 2,2-diméthyl 3-(1,2,2,2-tétrahaloéthyl) cyctopropane-1-carboxyliques, dans lesquels "halo" représente un atome de fluor, de chlore ou de brome, étant entendu que les copules acides et alcools des esters pyréthrinoïdes ci-dessus peuvent exister sous toutes leurs formes stéréoisomères possibles,

19. Les compositions et associations selon l'une quelconque des revendications 12 à 18 caractérisées en ce qu'elles renferment, en outre, un synergiste des pyréthrinoïdes.

## Patentansprüche

1. In sämtlichen ihrer möglichen isomeren Formen sowie die Gemische der Isomeren die Verbindungen der Formel (I)

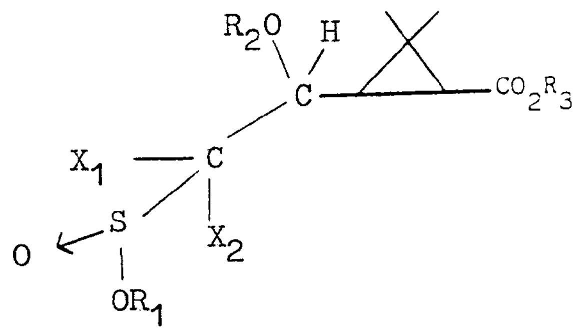

(I)

worin $R_1$ für ein Wasserstoffatom oder einen gesättigten oder unbesättigten, linearen, verzweigten oder cyclischen Kohlenwasserstoffrest mit bis zu 8 Kohlenstoffatomen steht, $R_2$ ein Wasserstoffatom, einen gesättigten oder ungesättigten, linearen, verzweigten oder cyclischen Kohlenwasserstoffrest mit bis zu 8 Kohlenstoffatomen oder einen gesättigten oder ungesättigten, linearen, verzweigten oder cyclischen Acylrest mit bis zu 9 Kohlenstoffatomen bedeutet, $X_1$ und $X_2$, die identisch oder voneinander verschieden sind, ein Fluor-, Chlor-, Brom- oder Jodatom, einen Rest $CF_3$, C≡N, $CO_2R$, worin R für einen linearen Alkylrest mit 1 bis 8 Kohlenstoffatomen steht, oder einen Rest

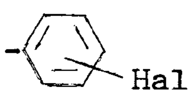

,

worin Hal für ein Fluor-, Chlor- oder Bromatom steht, wiedergeben und $R_3$ ein Wasserstoffatom, einen gesättigten, linearen Alkylrest mit bis zu 8 Kohlenstoffatomen oder

- entweder einen Benzylrest, gegebenenfalls substituiert durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxyrest und den Halogenatomen,
- oder eine Gruppe

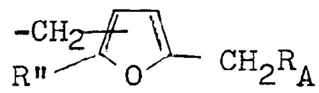

worin der Substituent R" ein Wasserstoffatom oder einen Methylrest bedeutet und der Substituent $R_A$ einen mono-cyclisden Arylrest oder eine Gruppe -C≡CH bedeutet, und insbesondere eine 5-Benzyl-3-furylmethylgruppe,

- oder eine Gruppe

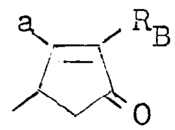

worin a ein Wasserstoffatom oder einen Methylrest bedeutet und Rg für einen aliphatischen, organischen Rest mit 2 bis 6 Kohlenstoffatomen und einer oder mehreren Kohlenstoff-Kohlenstoff-Unsättigungen und insbesondere den Rest $-CH_2-CH=CH_2$, $-CH_2-CH=CH-CH_3$, $-CH_2-CH=CH-CH=CH_2$, $-CH_2CH=CH-CH_2-CH_3$, $-CH_2-C≡CH$ steht,

- oder eine Gruppe

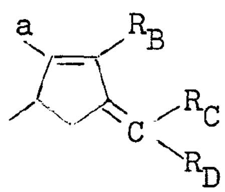

worin a und $R_B$ die vorstehend angegebene Bedeutung besitzen, $R_C$ und $R_D$, die identisch oder voneinander verschieden sind, ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyano-gruppe bedeuten,

- oder eine Gruppe

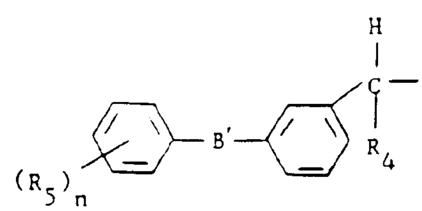

worin B' für ein Sauerstoff- oder Schwefelatom, eine Gruppe

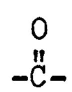

oder $-CH_2$ oder eine Sulfoxidgruppe oder eine Sulfongruppe steht und $R_4$ ein Wasserstoffatom, einen Rest -C≡N, eine Methylgruppe, einen Rest $-CONH_2$, einen Rest $-CSNH_2$ oder einen Rest -C≡CH bedeutet, $R_5$ ein Wasserstoffatom, ein Halogenaton oder einen Methylrest wiedergibt und n für eine Zahl entsprechend 0, 1 oder 2 steht, und insbesondere die 3-Phenoxylbenzyl-, α-Cyano-3-phenoxy-benzyl-, α-Ethinyl-3-phenoxybenzyl-, 3-Benzoylbenzyl-, 1-(3-Phenoxyphenyl)-ethyl- oder α-Thioamido-3-phenoxy-benzylgruppe,

- oder eine Gruppe

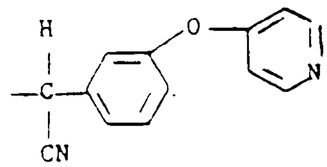

- oder eine Gruppe

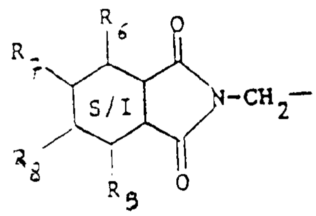

worin die Substituenten $R_6$, $R_7$, $R_8$ und $R_9$ ein Wasserstoffatom, ein Chloratom oder einen Methylrest bedeuten und worin S/I einen aromatischen Ring oder einen analogen Dihydro- oder Tetrahydroring symbolisiert,

- oder eine Gruppe

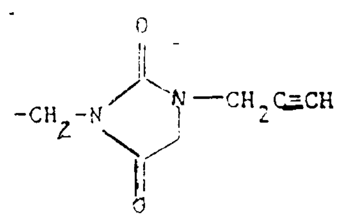

- oder eine Gruppe

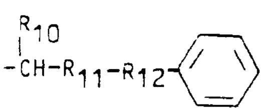

worin $R_{10}$ ein Wasserstoffatom oder einen Rest CN wiedergibt, $R_{12}$ einen Rest $-CH_2-$ oder ein Sauerstoffatom bedeutet, $R_{11}$ für einen Thiazolyl- oder Thiadiazolylrest steht, dessen Bindung mit

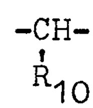

sich in irgendeiner der verfügbaren Positionen befinden kann, wobei $R_{12}$ an $R_{11}$ über das Kohlenstoffatom, das zwischen dem Schwefel- und einem Stickstoffatom liegt, gebunden ist,

- oder eine Gruppe

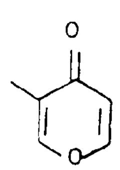

- oder eine Gruppe

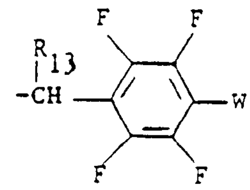

worin $R_{13}$ für ein Wasserstoffatom oder einen Rest CN steht und W ein Wasserstoffatom, ein Fluoratom, einen Methylrest oder eine Methoxygruppe bedeutet,

- oder eine Gruppe

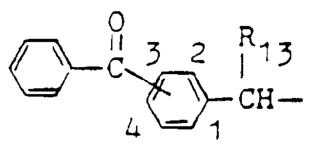

worin $R_{13}$wie vorstehend definiert ist und der Benzoylrest an die 3- oder 4-Stellungen des Phenylrestes gebunden ist,

- oder eine Gruppe

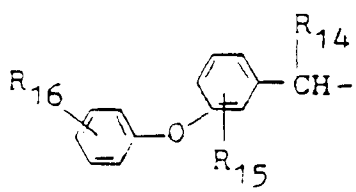

worin $R_{14}$ ein Wasserstoffatom, einen Methyl-, Ethinyl- oder Cyanorest bedeutet und $R_{15}$ und $R_{16}$, die voneinander verschieden sind, ein Wasserstoffatom, Fluor- oder Bromatom bedeuten,

- oder eine Gruppe

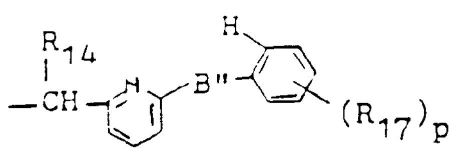

worin $R_{14}$ wie vorstehend definiert ist, ein jeder der Reste $R_{17}$ unabhängig ein Wasserstoffatom, eine Alkylgruppe mit 1 bis 4 Kohlenstoffatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen, Trifluormethyl, 3,4-Methylendioxy, Chlor, Fluor oder Brom bedeutet, p für eine Zahl entsprechend 0, 1 oder 2 steht und B" ein Sauerstoff- oder ein Schwefelatom wiedergibt,

- oder eine Gruppe

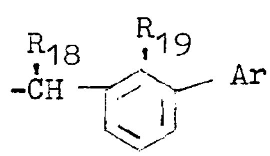

worin $R_{18}$ für ein Wasserstoffatom, einen Methyl-, Ethinyl- oder Cyanorest steht und $R_{19}$, verschieden, ein Wasserstoff-, Fluor- oder Bromatom bedeutet und Ar für einen Arylrest mit bis zu 14 Kohlenstoffatomen steht,

- oder eine Gruppe

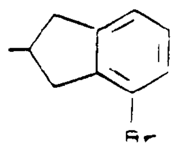

oder

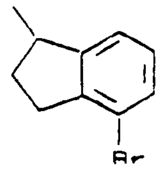

worin die Reste Ar wie vorstehend definiert sind, bedeutet.

2. Verbindungen der Formel I, wie in Anspruch 1 definiert, worin $X_1$ und $X_2$ jeweils ein Bromatom bedeuten.

3. Verbindungen der Formel I, wie in Anspruch 1 oder 2 definiert, worin $R_1$ ein Wasserstoffatom bedeutet.

4. Verbindungen der Formel I, wie in Anspruch 1 oder 2 definiert, worin $R_1$ einen Methylrest wiedergibt.

5. Verbindungen der Formel I, wie in einem der Ansprüche 1 bis 4 definiert, worin $R_2$ einen Rest $COCH_3$ bedeutet.

6. Verbindungen der Formel I, wie in einem der Ansprüche 1 bis 4 definiert, worin $R_2$ einen Methylrest bedeutet.

7. Verbindungen der Formel I, wie in einem der Anspruche 1 bis 6 definiert, worin die Cyclopropanverknüpfung die 1R,cis-Struktur besitzt.

8. Verbindungen der Formel I, wie in Anspruch 1 definiert, worin $R_3$ einen Rest

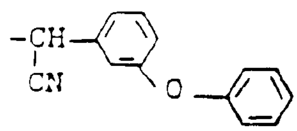

wiedergibt.

9. Verbindungen der Formel I, wie in Anspruch 8 definiert, worin $R_3$ einen Rest

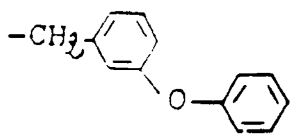

bedeutet.

10. Verfahren zur Herstellung der Verbindungen der Formel I, wie in einem der Ansprüche 1 bis 9 definiert, dadurch gekennzeichnet, daß man eine Verbindung der Formel (II)

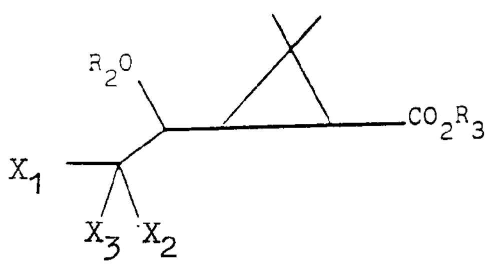

(II)

worin $X_1$, $X_2$, $R_2$ und $R_3$ die vorstehend angegebene Bedeutung besitzen und $X_3$ ein Chlor-, Brom- oder Jodatom wiedergibt, der Einwirkung von Natriumdithionit in wäßrigem Milieu unterzieht, um die einem Produkt der Formel (I) entsprechende Verbindung der Formel $I_A$ zu erhalten, worin $R_1$ ein Wasserstoffatom bedeutet,

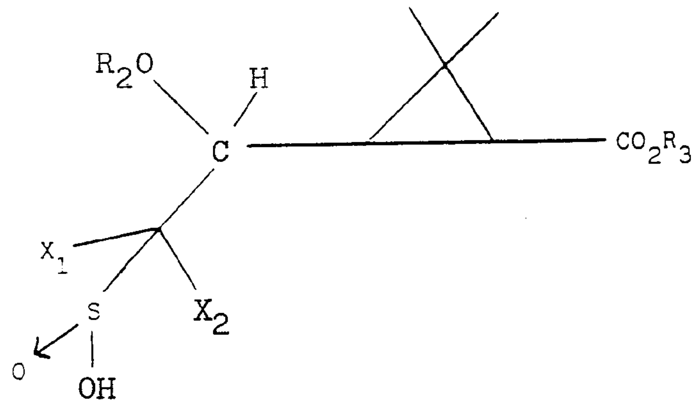

$(I_A)$

welche man gewünschtenfalls der Einwirkung eines Veretherungsmittels, das zur Einführung des Restes $R'_1$ befähigt ist, unterzieht, wobei $R'_1$ die Bedeutung von $R_1$ mit Ausnahme der Bedeutung Wasserstoff besitzt, um zu der Verbindung der Formel $I_B$

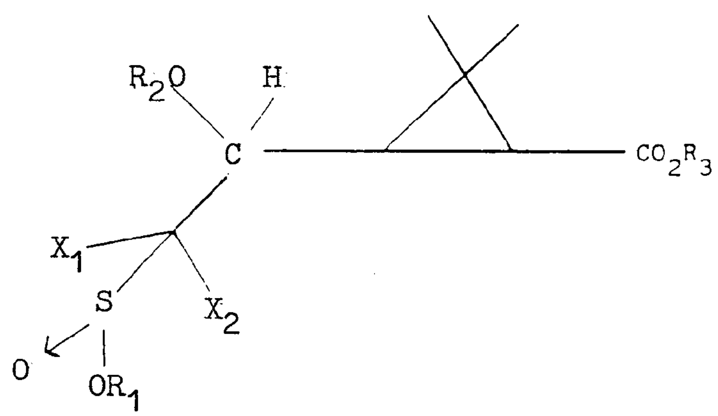

$(I_B)$

entsprechend einem Produkt der Formel (I), worin $R_1$ von einem Wasserstoffatom verschieden ist, zu gelangen.

**11.** Verwendung der Verbindungen der Formel I, wie in einem der Ansprüche 1 bis 9 definiert, bei der Bekämpfung von Parasiten der Pflanzen und Tiere und Parasiten von Räumlichkeiten.

**12.** Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eines der Produkte, wie in einem der Ansprüche 1 bis 9 definiert, enthalten.

**13.** Insektizide Zusammensetzungen, enthaltend als Wirkstoff zumindest eines der Produkte, wie in einem der Ansprüche 1 bis 9 definiert.

**14.** Akarizide Zusammensetzungen, enthaltend als Wirkstoff zumindest eines der Produkte, wie in einem der Ansprüche 1 bis 9 definiert.

**15.** Nematizide Zusammensetzungen, enthaltend als Wirkstoff zumindest eines der Produkte, wie in einem der Ansprüche 1 bis 9 definiert.

**16.** Akarizide Zusammensetzungen für die Bekämpfung von Parasiten warmblütiger Tiere, insbesondere von Zecken und Krätzmilben, dadurch gekennzeichnet, daß sie als Wirkstoff zumindest eines der Produkte, wie in einem der Ansprüche 1 bis 9 definiert, enthalten.

17. Zusammensetzungen für die tierische Ernährung, enthaltend als Wirkstoff zumindest eines der Produkte, wie in einem der Ansprüche 1 bis 9 definiert.

18. Assoziationen mit insektizider, akarizider oder nematizider Aktivität, dadurch gekennzeichnet, daß sie als Wirkstoff einesteils zumindest eine der Verbindungen der allgemeinen Formel (I) und anderenteils zumindest einen der Pyrethrinoidester, ausgewählt unter den Estern des Allethrolons, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der Chrysanthemumsäuren, unter den Estern des 5-Benzyl-3-furylmethylalkohols der 2,2-Dimethyl-3-(2-oxo-3,4,5,6-tetrahydrothiophenylidenmethyl)-cyclopropan-1-carbonsäuren, unter den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, unter den Estern der α-Cyano-3-phenoxybenzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-2-isopropylessigsäuren, unter den Estern der Allethrolone, des 3,4,5,6-Tetrahydrophthalimidomethylalkohols, des 5-Benzyl-3-furylmethylalkohols, des 3-Phenoxy-benzylalkohols und der α-Cyano-3-phenoxybenzylalkohole der 2,2-Dimethyl-3-(1,2,2,2-tetrahaloethyl)-cyclopropan-1-carbonsäuren, worin "halo" für ein Fluor-, Chlor- oder Bromatom steht, enthalten, mit der Maßgabe, daß die sauren Verknüpfungskomponenten und die Alkohole der vorstehenden Pyrethrinoidester in sämtlichen ihrer möglichen stereoisomeren Formen vorliegen können.

19. Zusammensetzungen und Assoziationen gemäß einem der Ansprüche 12 bis 18, dadurch gekennzeichnet, daß sie außerdem einen Synergisten der Pyrethrinoide enthalten.

## Claims

1. In all the possible stereoisomer forms, as well as isomer mixtures, the compounds corresponding to formula (I):

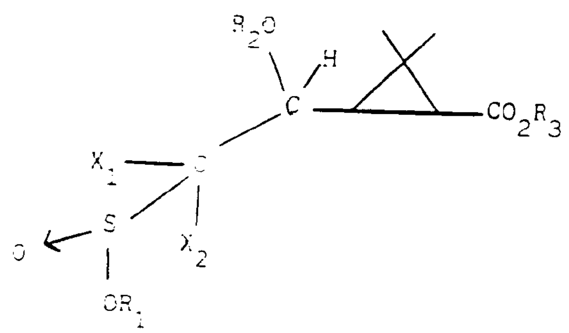

(I)

in which $R_1$ represents a hydrogen atom, or a saturated or unsaturated, linear, branched or cyclic hydrocarbon radical containing up to 8 carbon atoms, $R_2$ represents a hydrogen atom, a saturated or unsaturated, linear, branched or cyclic hydrocarbon radical containing up to 8 carbon atoms, or a saturated or unsaturated, linear, branched or cyclic acyl radical containing up to 9 carbon atoms, $X_1$ and $X_2$, identical or different, represent a fluorine, chlorine, bromine or iodine atom, a $CF_3$, C≡N, $CO_2R$ radical in which R represents a linear alkyl radical containing 1 to 8 carbon atoms or a

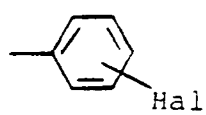

radical, in which Hal represents a fluorine, chlorine or bromine atom, and $R_3$ represents a hydrogen atom, a saturated linear alkyl radical containing up to 8 carbon atoms or:

- either a benzyl radical optionally substituted by one or more radicals chosen from the group constituted by alkyl radicals containing 1 to 4 carbon atoms, alkenyl radicals containing 2 to 6 carbon atoms, alkenyloxy radicals containing 2 to 6 carbon atoms, alkadienyl radicals containing 4 to 8 carbon atoms, the methylenedioxy radical and halogen atoms,

- or a group:

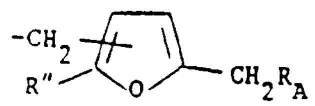

in which the R" substituent represents a hydrogen atom or a methyl radical and the $R_A$ substituent represents a monocyclic aryl or a -C≡CH group and in particular a 5-benzyl 3-furyl methyl group,

- or a group:

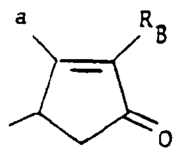

in which a represents a hydrogen atom or a methyl radical and $R_B$ represents an aliphatic organic radical containing 2 to 6 carbon atoms and one or more carbon-carbon unsaturations and in particular one of the following radicals: $-CH_2-CH=CH_2$, $-CH_2-CH=CH-CH_3$, $-CH_2-CH=CH-CH=CH_2$, $-CH_2-CH=CH-CH_2-CH_3$, $-CH_2-C≡CH$,

- or a group:

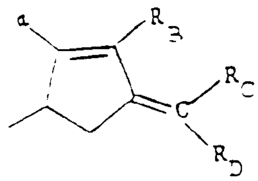

in which a and $R_B$ keep the same meaning as previously, $R_C$ and $R_D$, identical or different, represent a hydrogen atom, a halogen atom, an alkyl radical containing 1 to 6 carbon atoms, an aryl radical containing 6 to 10 carbon atoms, an alkyloxycarbonyl group containing 2 to 5 carbon atoms, or a cyano group,

- or a group:

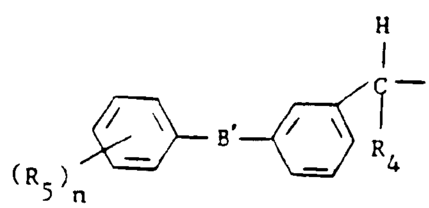

in which B' represents an oxygen or sulphur atom, a

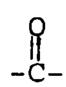

or $-CH_2$ group or a sulphoxide group or a sulphone group and $R_4$ represents a hydrogen atom, a -C≡N radical, a methyl radical, a $-CONH_2$ radical, a $-CSNH_2$ radical or a -C≡CH radical, $R_5$ represents a hydrogen atom, a halogen atom or a methyl radical and n represents a number equal to 0, 1 or 2, and in particular one of the following groups: 3-phenoxy benzyl, alpha-cyano 3-phenoxy benzyl, alpha-ethynyl 3-phenoxy benzyl, 3-benzoyl benzyl, 1-(3-phenoxy phenyl) ethyl or alpha-thioamido 3-phenoxy benzyl,

- or a group:

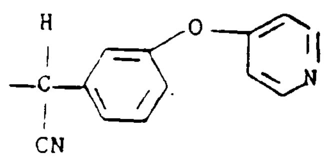

- or a group:

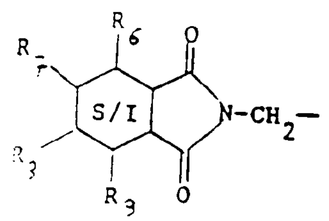

in which the $R_6$, $R_7$, $R_8$, $R_9$ substituents represent a hydrogen atom, a chlorine atom, or a methyl radical and in which S/I symbolizes an aromatic ring or a similar dihydro or tetrahydro ring,

- or a group:

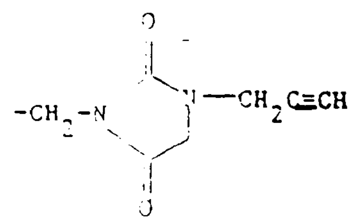

- or a group:

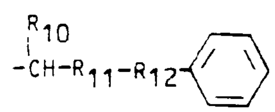

in which $R_{10}$ represents a hydrogen atom or a CN radical, $R_{12}$ represents a $-CH_2-$ radical or an oxygen atom, $R_{11}$ represents a thiazolyl or thiadiazolyl radical of which the bond with

$$-\underset{\displaystyle R_{10}}{\underset{|}{CH}}-$$

can be found in any of the available positions, $R_{12}$ being linked to $R_{11}$ by the carbon atom between the sulphur atom and a nitrogen atom,

- or a group:

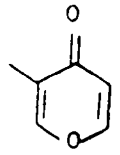

- or a group:

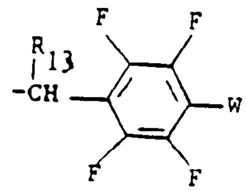

in which $R_{13}$ represents a hydrogen atom or a CN radical and W represents a hydrogen atom, a fluorine atom, a methyl radical or a methoxy radical,

- or a group

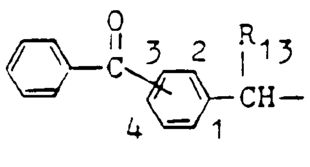

in which $R_{13}$ is defined as above, and the benzoyl radical is linked to position 3 or 4 of the phenyl,

- or a group:

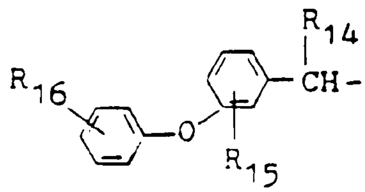

in which $R_{14}$ represents a hydrogen atom, a methyl, ethynyl or cyano radical and $R_{15}$ and $R_{16}$, being different, represent a hydrogen, fluorine or bromine atom,

- or a group:

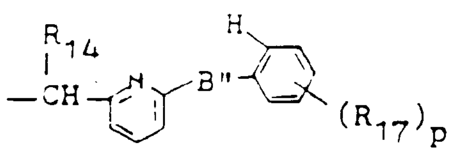

in which $R_{14}$ is defined as above, each of the $R_{17}$'s independently represents a hydrogen atom, an alkyl group containing 1 to 4 carbon atoms, an alkoxy group containing 1 to 4 carbon atoms, an alkylthio group containing 1 to 4 carbon atoms, an alkylsulphonyl group containing 1 to 4 carbon atoms, a trifluoromethyl, 3,4-methylenedioxy, chloro, fluoro or bromo group, p represents a number equal to 0, 1 or 2 and B" represents an oxygen atom or a sulphur atom,

- or a group:

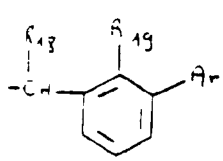

in which $R_{18}$ represents a hydrogen atom, a methyl, ethynyl or cyano radical and $R_{19}$, being different, represents a hydrogen, fluorine or bromine atom and Ar represents an aryl radical containing up to 14 carbon atoms,

- or a group:

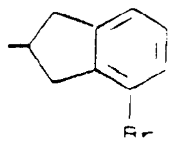

or

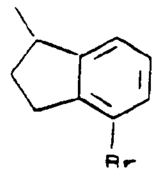

in which Ar is defined as previously.

2. The compounds of formula I as defined in claim 1 in which $X_1$ and $X_2$ each represents a bromine atom.

3. The compounds of formula I as defined in claim 1 or 2, in which $R_1$ represents a hydrogen atom.

4. The compounds of formula I as defined in claim 1 or 2, in which $R_1$ represents a methyl radical.

5. The compounds of formula I as defined in any one of claims 1 to 4 in which $R_2$ represents a $COCH_3$ radical.

6. The compounds of formula I as defined in any one of claims 1 to 4 in which $R_2$ represents a methyl radical.

7. The compounds of formula I as defined in any one of claims 1 to 6 in which the cyclopropane copula is of 1R cis structure.

8. The compounds of formula I defined in claim 1, in which $R_3$ represents a radical:

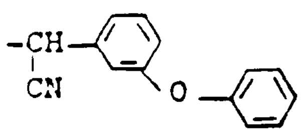

9. The compounds of formula I defined in claim 8, in which $R_3$ represents a radical:

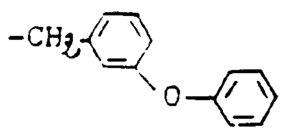

10. Preparation process for the compounds of formula I as defined in any one of claims 1 to 9, characterized in that a compound of formula (III):

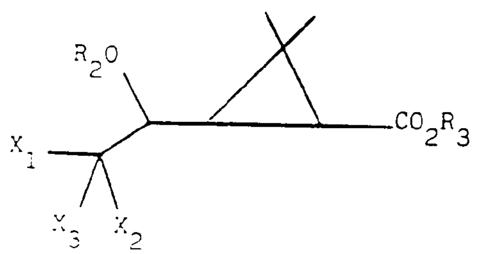

(III)

in which $X_1$, $X_2$, $R_2$ and $R_3$ keep the same meaning as previously and $X_3$ represents a chlorine, bromine or iodine atom, is subjected to the action of sodium dithionite in an aqueous medium in order to obtain the compound of formula ($I_A$) corresponding to a product of formula (I) in which $R_1$ represents a hydrogen atom

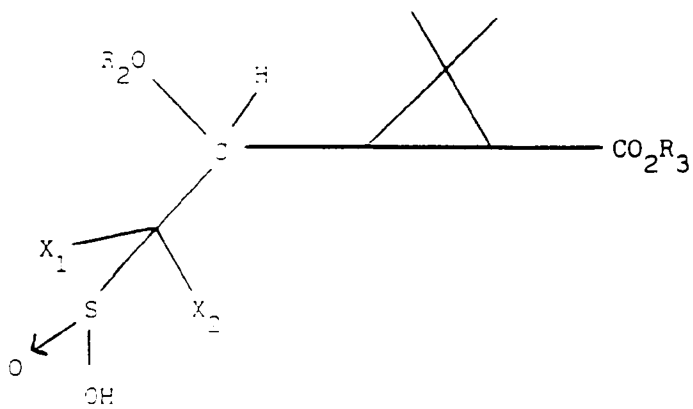

($I_A$)

which is subjected, if desired, to the action of an etherification agent capable of introducing the R'$_1$ radical, R'$_1$ having the meaning of $R_1$ with the exception of the hydrogen value, in order to obtain the compound of formula $I_B$:

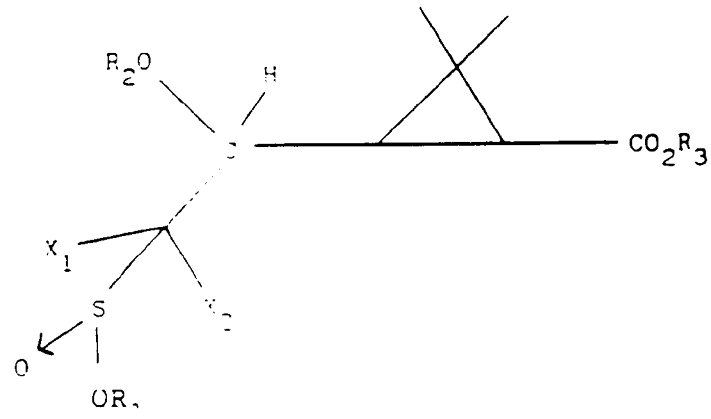

($I_B$)

corresponding to a product of formula (I) in which $R_1$ is different from a hydrogen atom.

11. Use of the compounds of formula (I) as defined in one of claims 1 to 9, for combating parasites of vegetation and animals and parasites of premises.

12. The compositions intended for combating parasites of vegetation, parasites of premises and parasites of warm-blooded animals, characterized in that they contain as active ingredient at least one of the products defined in any one of claims 1 to 9.

13. The insecticide compositions containing as active ingredient at least one of the products defined in any one of claims 1 to 9.

14. The acaricide compositions containing as active ingredient at least one of the products defined in any one of claims 1 to 9.

15. The nematicide compositions containing as active ingredient at least one of the products defined in any one of claims 1 to 9.

16. The acaricide compositions intended for combating parasites of warm-blooded animals, in particular ticks and mites, characterized in that they contain as active ingredient at least one of the products defined in any one of claims 1 to 9.

17. The compositions intended for animal feed containing as active ingredient at least one of the products defined in any one of claims 1 to 9.

18. Combinations endowed with insecticide, acaricide or nematicide activity, characterized in that they contain as active ingredient, on the one hand at least one of the compounds of general formula (I) and on the other hand, at least one of the pyrethrinoid esters chosen from the group constituted by the esters of allethrolone, of 3,4,5,6-tetrahydrophthalimidomethyl alcohol, of 5-benzyl-3-furyl methyl alcohol, of 3-phenoxybenzyl alcohol and of alpha-cyano-3-phenoxybenzyl alcohols with chrysanthemic acids, by the esters of 5-benzyl-3-furyl methyl alcohol with 2,2-dimethyl-3-(2-oxo-3,4,5,6-tetrahydrothiophenylidenemethyl)-cyclopropane-1-carboxylic acids, by the esters of 3-phenoxybenzyl alcohol and of alpha-cyano-3-phenoxybenzyl alcohols with 2,2-dimethyl-3-(2,2-dichlorovinyl)cyclopropane-1-carboxylic acids, by the esters of alpha-cyano-3-phenoxy-benzyl alcohols with 2,2-dimethyl-3-(2,2-dibromovinyl)-cyclopropane-1-carboxylic acids, by the esters of 3-phenoxybenzyl alcohol with 2-parachlorophenyl-2-isopropyl acetic acids, by the esters of allethrolone, of 3,4,5,6-tetrahydrophtalimidomethyl alcohol, of 5-benzyl-3-furyl methyl alcohol, of 3-phenoxybenzyl alcohol, and of alpha-cyano-3-phenoxybenzyl alcohols with 2,2-dimethyl-3-(1,2,2,2-tetrahaloethyl)-cyclopropane-1-carboxylic acids, in which "halo" represents a fluorine,chlorine or bromine atom, it being understood that the acid and alcohol copulas of the above pyrethrinoid esters can exist in all their possible stereoisomer forms.

**19.** The compositions and combinations according to any one of claims 12 to 18 characterized in that they contain, in addition, a pyrethrinoid synergist.